# EUROPEAN PATENT APPLICATION

(11) **EP 4 649 985 A2**
(43) Date of publication of application: **19.11.2025**
(21) Application number: 25207031.3
(22) Date of filing: 16.03.2023
(51) Int. Cl.: A61M 16/00

(54) **OXYGEN FEEDBACK CONTROL OF HIGH FLOW NASAL CANNULA DEVICE**

(30) Priority: 17.03.2022 US 202263320921 P; 23.02.2023 US 202318113339
(62) Divisional of application: 23162397.6
(71) Applicant: Hill-Rom Services PTE. LTD., Singapore 768923 (SG)
(72) Inventor: BARILEA, Deny Duay, 768923 Singapore (SG); LIEW, Justin Xuan Kai, 768923 Singapore (SG); WU, Baoyi, 768923 Singapore (SG)
(74) Representative: Reddie & Grose LLP

(57) **Abstract**

A high-flow respiratory therapy system includes a blender arranged to receive a first gas and a second gas and to output a combination thereof as a delivered gas to a patient respiratory interface, an airflow source for providing a flow of air to the blender as the first gas, a valve operable to provide oxygen gas from an oxygen gas source to the blender as the second gas, a heater operable to heat the delivered gas at the patient respiratory interface, a pulse oximeter, and a controller configured to execute a learning procedure in response to a trigger. The learning procedure may include varying a parameter of the airflow source, a parameter of the valve, and a parameter of the heater and determining a recommended parameter based on one or more measurements of the pulse oximeter. The controller may output a recommendation to adjust the recommended parameter.

## Description

The efficiency of high-flow respiratory therapy, both in terms of the clinician's time spent and the oxygen that is used up, is limited by the technology available to conventional high-flow devices. In general, high-flow respiratory therapy may be characterized by the delivery of a specified fraction of inspired oxygen (FiO2) at a flow rate equal to or greater than the inspiratory flow rate of the patient (so that the specified FiO2 is not diluted by ambient air as it would be if the patient's inspiratory flow rate were to exceed the delivered flow rate). As the patient's respiratory needs change during treatment, either or both of the flow rate and the specified FiO2 may need to be adjusted in order for the therapy to be as beneficial as possible to the patient. However, current high-flow devices require the clinician to manually make adjustments to the settings of the device to change the specified FiO2, the flow rate, or other parameters based solely on the clinician's own calculations and expertise.

The present disclosure contemplates various systems and methods for overcoming the above drawbacks accompanying the related art. One aspect of the embodiments of the present disclosure is a high-flow respiratory therapy system. The high-flow respiratory therapy system may comprise a blender arranged to receive a first gas and a second gas and to output a combination of the first gas and the second gas as a delivered gas to a patient respiratory interface, an airflow source for providing a flow of air to the blender as the first gas, a valve operable to provide oxygen gas from an oxygen gas source to the blender as the second gas, a heater operable to heat the delivered gas at the patient respiratory interface, a pulse oximeter, and a controller configured to execute a learning procedure in response to a trigger. The learning procedure may comprise varying a first parameter of the airflow source, a second parameter of the valve, and a third parameter of the heater and determining a recommended parameter from among the first, second, and third parameters based on one or more measurements of the pulse oximeter. The controller may be further configured to output a recommendation to adjust the recommended parameter.

The varying of the first parameter, the second parameter, and the third parameter may include performing a series of experimental runs. Each of the runs may include varying one or more of the first, second, and third parameters and recording a resulting measurement of the pulse oximeter. The determining of the recommended parameter may include comparing the recorded measurements of the pulse oximeter.

The trigger may comprise a passage of a predefined length of time. The trigger may occur periodically according to the predefined length of time. The trigger may comprise a predefined measurement of the pulse oximeter. The trigger may comprise a predefined degree of change in a measurement of the pulse oximeter. The trigger may comprise a manually entered command.

The controller may be configured to output the recommendation as a visual indication on a display. The recommendation may comprise a direction in which to adjust the recommended parameter. The recommendation may comprise an amount by which to adjust the recommended parameter.

The controller may be configured to plot a plurality of measurements of the pulse oximeter as a function of time on a display.

The high-flow respiratory therapy system may comprise a flow sensor arranged to measure a flow rate of the delivered gas, an oxygen sensor arranged to measure a fraction of inspired oxygen (FiO2) of the delivered gas, and a temperature sensor arranged to measure a temperature of the delivered gas at the patient respiratory interface.

The high-flow respiratory therapy system may comprise a humidification system for humidifying the delivered gas as it flows from the blender to the patient respiratory interface. The high-flow respiratory therapy system may comprise a second heater operable to heat the delivered gas upstream of the humidification system.

The airflow source may comprise a blower. The airflow source may comprise a compressed gas source.

Another aspect of the embodiments of the present disclosure is a method of controlling a high-flow respiratory therapy system. The method may comprise receiving a trigger and executing a learning procedure in response to the trigger. The learning procedure may comprise varying a first parameter of an airflow source that provides a flow of air to a blender of the high-flow respiratory therapy system and varying a second parameter of a valve operable to provide oxygen gas from an oxygen gas source to the blender, the blender being arranged to receive the flow of air from the airflow source as a first gas, receive the oxygen gas from the valve as the second gas, and output a combination of the first gas and the second gas as a delivered gas to a patient respiratory interface. The learning procedure may further comprise varying a third parameter of a heater operable to heat the delivered gas at the patient respiratory interface and determining a recommended parameter from among the first, second, and third parameters based on one or more measurements of a pulse oximeter. The method may further comprise outputting a recommendation to adjust the recommended parameter.

Another aspect of the embodiments of the present disclosure is a method of providing high-flow respiratory therapy to a patient. The method may comprise the above method of controlling a high-flow respiratory therapy system, wherein the patient respiratory interface is connected to the patient. The outputting of the recommendation may comprise presenting the recommendation on a graphical user interface. The method of providing high-flow respiratory therapy to the patient may further comprise receiving a user input to the graphical user interface and adjusting the recommended parameter in response to the user input.

Another aspect of the embodiments of the present disclosure is a non-transitory program storage medium on which are stored instructions executable by a processor or programmable circuit to perform operations for controlling a high-flow respiratory therapy system. The operations may comprise receiving a trigger and executing a learning procedure in response to the trigger. The learning procedure may comprise varying a first parameter of an airflow source that provides a flow of air to a blender of the high-flow respiratory therapy system and varying a second parameter of a valve operable to provide oxygen gas from an oxygen gas source to the blender, the blender being arranged to receive the flow of air from the airflow source as a first gas, receive the oxygen gas from the valve as the second gas, and output a combination of the first gas and the second gas as a delivered gas to a patient respiratory interface. The learning procedure may further comprise varying a third parameter of a heater operable to heat the delivered gas at the patient respiratory interface and determining a recommended parameter from among the first, second, and third parameters based on one or more measurements of a pulse oximeter. The operations may further comprise outputting a recommendation to adjust the recommended parameter.

The invention will now be further described by way of example with reference to the accompanying drawings, in which:
Figure 1 shows an exemplary high-flow respiratory therapy system according to an embodiment of the present disclosure;
Figure 2 shows an exemplary data structure that may be used by the high-flow respiratory therapy system to execute a learning procedure for adjusting one or more parameters of the delivered gas;
Figure 3 shows an exemplary graphical user interface of the high-flow respiratory therapy system;
Figure 4 is a graphical representation of exemplary moisture saturation curves of the delivered gas;
Figure 5 is an exemplary operational flow according to an embodiment of the present disclosure; and
Figure 6 is an exemplary sub-operational flow of step 520 in Figure 5.

The present disclosure encompasses various embodiments of high-flow respiratory therapy systems and associated methods. The detailed description set forth below in connection with the appended drawings is intended as a description of several currently contemplated embodiments and is not intended to represent the only form in which the disclosed invention may be developed or utilized. The description sets forth the functions and features in connection with the illustrated embodiments. It is to be understood, however, that the same or equivalent functions may be accomplished by different embodiments that are also intended to be encompassed within the scope of the present disclosure. It is further understood that the use of relational terms such as first and second and the like are used solely to distinguish one from another entity without necessarily requiring or implying any actual such relationship or order between such entities.

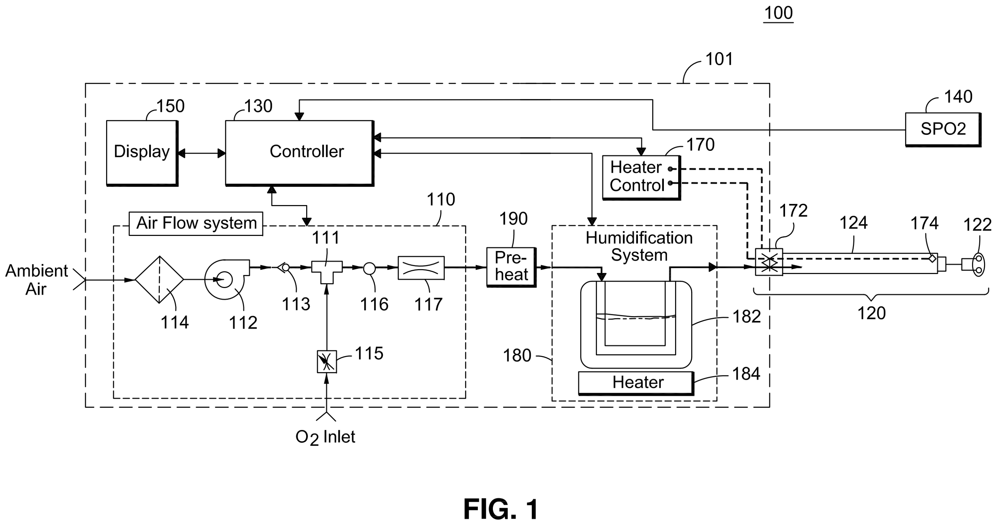

Figure 1 shows an exemplary high-flow respiratory therapy system 100 according to an embodiment of the present disclosure. The high-flow respiratory therapy system 100 may be used to deliver high-flow oxygen therapy to a patient experiencing hypoxemic respiratory insufficiency caused by various conditions such as chronic obstructive pulmonary disease (COPD) and acute respiratory distress syndrome (ARDS), including those patients suffering from viral infections and resulting pneumonia associated with the ongoing pandemic of coronavirus disease 2019 (COVID-19). In use, a prescribed mixture of ambient air and oxygen gas may be delivered by an air flow system 110 to a patient respiratory interface 120 comprising a nasal cannula 122 worn by the patient. Unlike conventional high-flow devices, the system 100 may include a controller 130 that is configured to automatically recommend adjustments to various parameters of the therapy. In particular, the controller 130 may execute a learning procedure that involves varying each parameter based on the patient's oxygen saturation (SpO2) as measured by a pulse oximeter 140 (which may communicate with the controller 130 by Bluetooth, for example). The controller 100 may output the resulting recommendation to a display 150, and the clinician can then make the needed adjustment manually based on the recommendation. In some iterations, the controller 100 may automatically make the recommended adjustment without the clinician's manual input. In either case, the clinician need not rely solely on his/her own determination of which parameters to adjust, saving the clinician's time while greatly improving the quality of the analysis that goes into each adjustment. The resulting therapy may be more beneficial to the patient while at the same time making more efficient use of limited resources such as oxygen gas.

In order to provide the patient with a specified fraction of inspired oxygen (FiO2), the air flow system 110 may include a blender 111 arranged to receive a first gas (e.g., ambient air) and a second gas (e.g., O₂ gas) and to output a combination thereof as a delivered gas to the patient respiratory interface 120. An airflow source 112 such as a blower or compressed gas source may provide a flow of air to the blender 111 as the first gas, via a check valve 113 for example. In the case of the illustrated air flow system 110, for example, ambient air from the room may pass through a filter 114 before being elevated to a higher flow rate by a blower 112 and provided to the blender 111 as the first gas. The second gas (e.g., O₂ gas) may arrive at the blender 111 via a valve 115 (e.g., a proportional valve) connected to an oxygen gas source such as an oxygen cannister or O₂ concentrator (e.g., a portable oxygen concentrator), for example. The settings of the airflow source 112 and valve 115 may thus determine the FiO2 and flow rate of the delivered gas output by the blender 111, which may be measured respectively by an O₂ sensor 116 and a flow sensor 117 of the air flow system 110.

The learning procedure executed by the controller 130 may comprise varying a first parameter of the airflow source 112 such as a blower speed in the case of a blower or a valve setting in the case of a compressed gas source, for example, in order to adjust the flow rate of the ambient air. The learning procedure may further comprise varying a second parameter of the valve 115 in order to adjust the amount of oxygen gas that is included in the delivered gas. In addition, the learning procedure may comprise varying a third parameter of a heater 170 that is operable to heat the delivered gas at the patient respiratory interface 120 (e.g., via a heating element 172 disposed in communication with a gas delivery conduit 124 of the patient ventilation interface 120). Each of the first, second, and third parameters may be varied by a small amount, for example, one that is substantial enough to produce a measurable change in the patient's SpO2 but not substantial enough to cause a change in the therapeutic effect of the treatment to the patient. By varying the parameters in this way individually and/or in different combinations, the controller 100 may learn which parameter or combination of parameters has the most significant effect on the patient's SpO2 at any given moment during the therapy. The controller 110 may then identify the most significant parameter as the recommended parameter to be adjusted. Thus, for example, in a case where purging the patient's anatomical dead space would have a greater effect on patient oxygenation than changing the delivered FiO2 (e.g., due to nasopharyngeal airway resistance), the controller 110 may determine that oxygenation can be most efficiently improved by increasing total airflow and/or adjusting the temperature of the delivered gas rather than wastefully pumping more oxygen as in conventional devices.

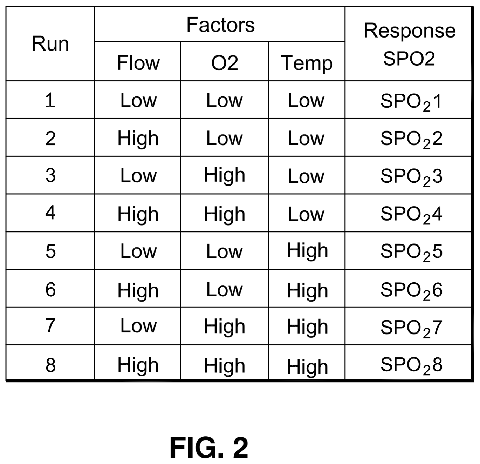

Figure 2 shows an exemplary data structure that may be used by the high-flow respiratory therapy system 100 to execute the learning procedure. The data structure is illustrated in tabular form to represent how the learning data may be organized and/or indexed, though the particular form of the data structure is not intended to be limited in this respect. By way of example, the data structure of Figure 2 may represent a single iteration of an exemplary learning procedure consisting of eight experimental runs (numbered 1 through 8) performed in rapid succession by the controller 130. In each run, the controller 130 may adjust one or more of first ("Flow"), second ("O₂"), and third ("Temp") parameters or factors and record a response SpO2 of the pulse oximeter 140. For the sake of simplicity, the first and second parameters may be thought of as corresponding generally to a total flow and an FiO2. However, it is noted that the first and second parameters, if defined as a parameter of the airflow source 112 and a parameter of the valve 115, may not correspond exactly to a total flow and an FiO2 as these parameters may have some codependence depending on the architecture of the air flow system 110 and, in particular, the positions of the oxygen sensor 116 and flow sensor 117 (since adjusting the valve 115 to increase the O₂ gas introduced to the blender 111 would also slightly increase the total flow measured by the flow sensor 117, for example).

As shown in Figure 2, each of the first, second, and third parameters, may be adjusted in each run by a "Low" or a "High" amount, with the recorded SpO2 measurements (SPO₂1, SPO₂2, ... SPO₂8) being indicative of the effect of adjusting the parameter(s) by the "High" amount. The "Low" amount may be approximately zero or no adjustment, for example, and the "High" amount may be the small amount described above. Thus, in the illustrated example, Run 1 may record a response SPO₂1 to no change or no substantial change (e.g., a control run), Run 2 may record a response SPO₂2 to the first parameter ("Flow") being adjusted by a small amount, Run 3 may record a response SPO₂3 to the second parameter ("O2") being adjusted by a small amount, Run 4 may record a response SPO₂4 to the first parameter ("Flow") and the second parameter ("O2") being adjusted by small amounts, Run 5 may record a response SPO₂5 to the third parameter ("Temp") being adjusted by a small amount, Run 6 may record a response SPO₂6 to the first parameter ("Flow") and the third parameter ("Temp") being adjusted by small amounts, Run 7 may record a response SPO₂7 to the second parameter ("O2") and the third parameter ("Temp") being adjusted by small amounts, and Run 8 may record a response SPO₂8 to the first parameter ("Flow"), the second parameter ("O2"), and the third parameter ("Temp") being adjusted by small amounts. The directions of the small adjustments may be determined by the controller 130 according to the direction that the SpO2 is needed to be changed. So, for example, if the trigger for initiating the learning procedure is a drop in SpO2, then the controller 130 may make the small adjustments in a direction tending to increase the SpO2.

Based on the plurality of recorded SpO2 (SPO₂1, SPO₂2, ... SPO₂8), the controller 130 may determine an optimal one or more of the parameters to be the recommended parameter for adjustment. This may be the parameter or combination of parameters that resulted in the greatest response SpO2, for example. As another possibility, the recommended parameter may be the single parameter that resulted in the greatest response SpO2 (e.g., from among Runs 2, 3, and 5), discounting runs in which more than one parameter is adjusted. Selecting a single parameter may be beneficial from the standpoint of efficiency since adjusting a combination of parameters (e.g., three parameters as in Run 8) might inefficiently waste power or oxygen despite resulting in a greater response SpO2. In some cases, the controller 130 might score each combination of parameters according to the size of the response SpO2 with the score being weighted, penalized, or otherwise modified based on the efficiency or inefficiency of the adjustment. For example, the response SPO₂8 might be greater than the response SpO2 of another run but might be scored lower because it uses more oxygen gas to achieve only a slightly greater response SpO2. The controller 130 may then recommend the combination of one or more parameters corresponding to the run with the greatest score, sometimes recommending a single parameter and other times recommending a combination of parameters.

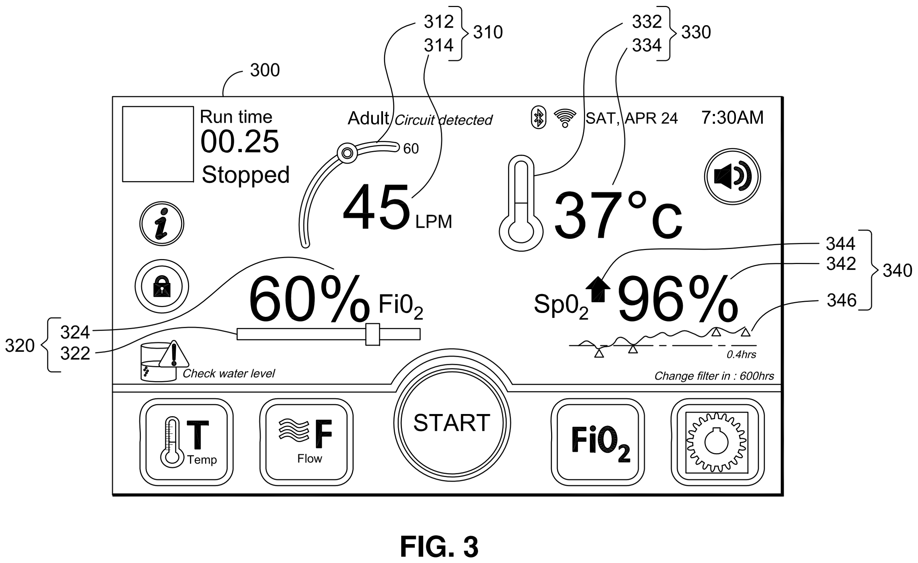

Figure 3 shows an exemplary graphical user interface (GUI) 300 of the high-flow respiratory therapy system 100. The GUI 300 may be presented on the display 150 (*see* Figure 1), which may be at least partially within the same housing 101 of the system 100 that contains the air flow system 110 and controller 130, for example. The display 150 may comprise a touchscreen (e.g., a 5" LCD) that faces outward from the housing 101, for example, to allow the clinician (or patient) to interact with the GUI 300 to adjust settings directly using the display 150. Alternatively, or additionally, interaction with the GUI 300 may be via softkeys or other buttons, dials, knobs, switches, etc. that are provided on the housing 101 adjacent the display 150. The exemplary GUI 300 shown in Figure 3 provides an example of how a clinician may efficiently and easily adjust the parameters of the therapy in accordance with the recommendation generated by the controller 130. In this regard, the GUI 300 may include a flow control portion 310, an FiO2 control portion 320, a temperature control portion 330, and an SpO2 feedback portion 340. The flow control portion 310 may allow the user (e.g., clinician) to make adjustments to the total flow of gas delivered by the air flow system 110 to the patient respiratory interface 120 and, in particular, to a parameter of the airflow source 112 (e.g., blower motor speed) referred to herein as the first parameter. The flow control portion 310 may include a flow control tool 312 such as a slider, dial, arrow keys, etc. for increasing or decreasing the total flow and a total flow display 314 indicating the current setting (e.g., 45 LPM) as may be measured by the flow sensor 117. When the user wishes to adjust the total flow of the delivered gas, he/she may do so using the flow control tool 312 (e.g., by sliding his/her finger on the touchscreen) to change the total flow to the desired setting as indicated by the total flow display 314. The FiO2 control portion 320 may correspondingly allow the user to make adjustments to the FiO2 of the delivered gas (e.g., to a parameter of the valve 115, referred to as the second parameter herein) and may similarly include an FiO2 control tool 322 (e.g., slider) and an FiO2 display 324 indicating the current setting (e.g., 60%) as may be measured by the oxygen sensor 116. The temperature control portion 330 may correspondingly allow the user to make adjustments to the temperature of the delivered gas (e.g., to a parameter of the heater 170, referred to as the third parameter herein) and may similarly include a temperature control tool 332 (e.g., slider) and a temperature display 334 indicating the current setting (e.g., 37 °C) as may be measured by a temperature sensor 174 in the patient interface 120 (e.g., in the gas delivery conduit 124 thereof). As noted above, the measured total flow or FiO2 (or the measured temperature) may in fact depend on more than one of these three parameters, in which case the values indicated by each of the displays 314, 324, 334 may to some extent depend on the combination of settings as adjusted by the user.

The SpO2 feedback portion 340 may display information about the patient's SpO2 as measured by the pulse oximeter 140 (which may be attached to the patient's body, for example). In the example shown in Figure 3, the SpO2 feedback portion 340 includes a current SpO2 display 342 indicating the current SpO2 as a percentage (e.g., 96%), an SpO2 trend indicator 344 (e.g., upward arrow) showing the current direction of change in SpO2 (e.g., the sign of the first order derivative with respect to time), and an SpO2 history indicator 346 showing historical values of the patient's SpO2 over a period of time (e.g., "0.4hrs") leading up to the present. The historical values may be plotted as a line graph as shown, for example, with the x-axis representing time and the y-axis representing SpO2 level. The SpO2 history indicator 346 may include pointers (e.g., small triangles in Figure 3) indicating when changes were made to the first/second/third parameters or other settings of the high-flow respiratory therapy system 100. In this way, the user may better understand the likely causes of a given trend or change in the SpO2 level of the patient, noting, for example, that a recent rise in SpO2 followed an adjustment to the parameters of the therapy. It is contemplated, for example, that the user may be able to tap or otherwise interact with a given pointer to drill down on the event and see which parameters were adjusted, in which direction, by how much, etc.

The GUI 300 may have various additional features beyond those described above, some of which are illustrated in the example shown in Figure 3. For example, the high-flow respiratory system 100 may have the capability to detect and/or monitor whether the patient interface 120 is connected, in use, etc. and in some cases which size or type of patient interface 120 is being used (e.g., using a digital signal read from the patient interface 120 by the controller 130). In this respect, the GUI 300 may include an indication of which patient interface 120 is detected, which may read "Adult Circuit detected" as shown in Figure 3, referring to a patient interface 120 or nasal cannula 122 sized for an adult patient (as opposed to a child), for example.

Referring back to Figure 1, the high-flow respiratory system 100 may include a humidification system 180 for humidifying the delivered gas as it flows from the blender 111 of the air flow system 110 to the patient respiratory interface 120. The humidification system 180 may comprise a moisture source 182 such as a water tank and associated tubing and a heater 184 arranged to heat the water contained in the moisture source 182. As the delivered gas flows through the humidification system 180, water evaporating from the heated moisture source 182 may enter the delivered gas, increasing its humidity so that it is not too dry for the patient's airway. In one preferred embodiment, the delivered gas may pass through the moisture source 182 in the form of bubbles flowing through the heated water. This may have the beneficial effect of increasing the surface area for evaporation as the water evaporates into the delivered gas on the surface of each bubble. However, other types of humidification systems such as passover humidifiers that might not make use of the heater 184 are also contemplated herein.

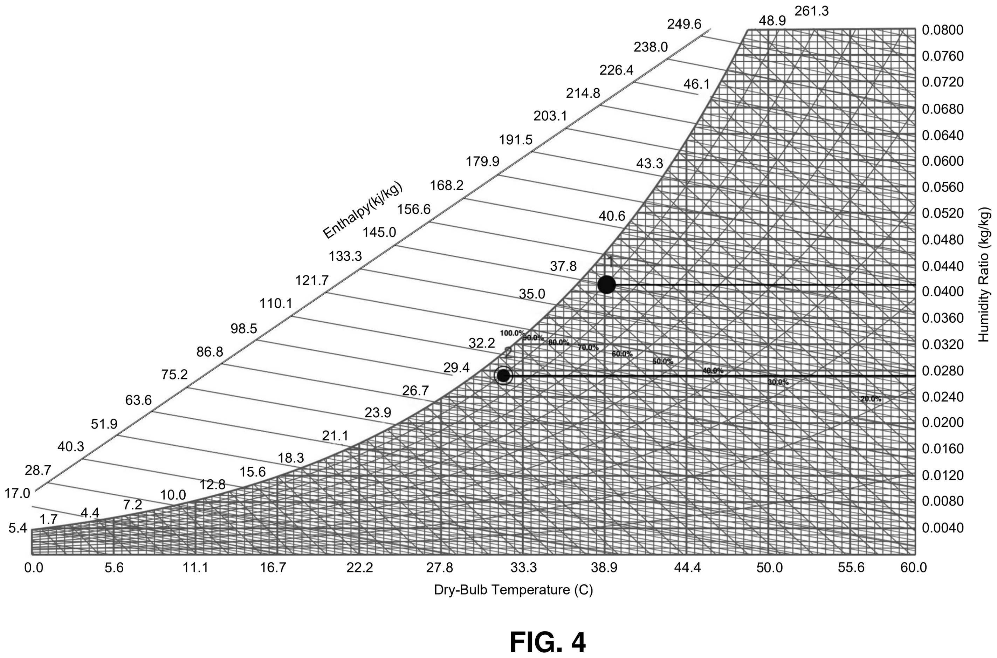

Figure 4 is a graphical representation of exemplary moisture saturation curves of the delivered gas output from the air flow system 110 to the patient respiratory interface 120. As can be seen from a comparison of two points in Figure 4, more moisture content is possible when the delivered gas is at a higher temperature. For example, the following Table 1 describes the two indicated points in Figure 4:

**Table 1**

| | | |
|---|---|---|
| Dry-Bulb Temp (x-axis) | 39.0 °C | 32.0 °C |
| Humidity Ratio (y-axis) | 0.0411 kg/kg | 0.0272 kg/kg |
| Relative Humidity | 89.5% | 89.2% |
| Wet-Bulb Temp | 37.3 °C | 30.4 °C |
| Partial Pressure | 6.276 kPa abs | 4.251 kPa abs |
| Enthalpy | 144.9 kJ/kg | 101.9 kJ/kg |
| Specific Volume | 0.943 m³/kg | 0.902 m³/kg |
| Dew Point Temp | 37.0 °C | 30.0 °C |

In particular, for substantially the same relative humidity of around 90%, the warmer gas (dry-bulb temperature = 39.0 °C) has significantly more moisture content (humidity content = 0.0411 kg/kg) as compared to the cooler gas (dry-bulb temperature = 32.0 °C, humidity content = 0.272 kg/kg). Therefore, in order to increase the moisture content of the delivered gas, it is contemplated that the high-flow respiratory therapy system 100 may include a heater 190 that is operable to heat the delivered gas upstream of the humidification system 180. The heater 190 may be arranged downstream of the air flow system 110 (e.g., downstream of the blender 111 or of a subsequent oxygen sensor and/or flow sensor 117) and upstream of the moisture source 182 of the humidification system 180, for example. By pre-heating the delivered gas in this way, it is envisioned that the moisture content added by the humidification system 180 will be greater, resulting in improved comfort to the patient undergoing the high-flow respiratory therapy and more efficient use of the humidification system 180.

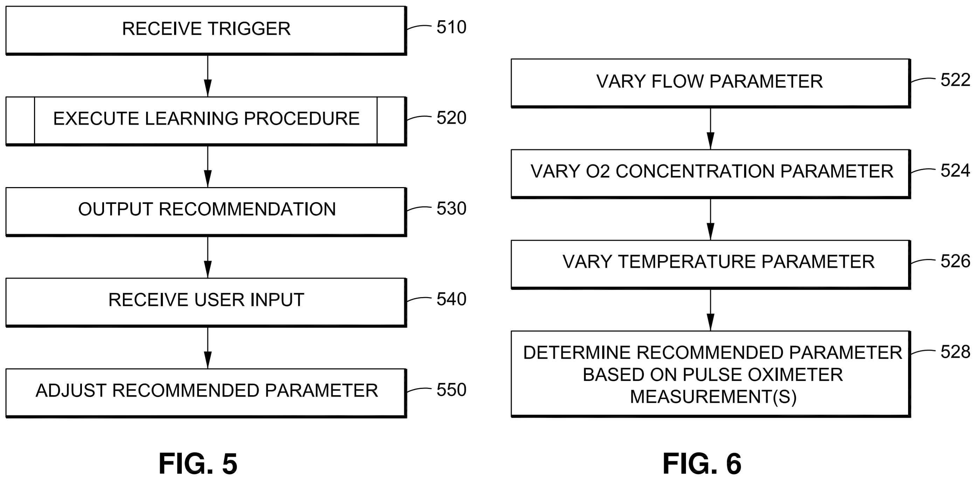

Figure 5 is an exemplary operational flow according to an embodiment of the present disclosure. The operational flow of Figure 5 may be performed by a high-flow respiratory therapy system such as the system 100 shown in Figure 1 and described above in relation to Figures 1-4. Referring to the disclosed features of the system 100 by way of example, the operational flow of Figure 5 may begin with receiving a trigger for initiating a learning procedure (step 510). For example, the controller 130 may receive the trigger in the form of a combination of sensor input from any or all of the oxygen sensor 116, flow sensor 117, temperature sensor 174, and pulse oximeter 140, clock data from an internal clock of the system 100, and/or a command originating external to the system 100, such as a user input or instruction from a remote server. More specifically, the contemplated trigger may comprise the passage of a predefined length of time, such as a time since the initiation of therapy (e.g., one minute into therapy, one hour into therapy, etc.), a time since a previous trigger, or a time since any other event (e.g., SpO2 measurement and/or other sensor readings). The trigger may, for example, occur periodically according to the predefined length of time (e.g., every minute, every ten seconds, etc.). The trigger may instead or additionally comprise a predefined measurement of the pulse oximeter 140 or other sensor. For example, when the patient's SpO2 as measured by the pulse oximeter 140 dips below a predefined threshold, the controller 130 may be triggered to initiate the learning procedure so that the parameters of the therapy can be readily and quickly adjusted to correct the drop in SpO2 for the sake of the patient's health. Along the same lines, the trigger may comprise a predefined degree of change in a measurement of the pulse oximeter 140, such as a first order derivative of the SpO2 with respect to time. This may allow for a more rapid response by the controller 100 since the trigger may be received at a time when the SpO2 is only predictive of a drop (e.g., when a negative slope of the SpO2 exceeds a threshold) rather than when the SpO2 itself has already crossed a threshold. The trigger may also comprise a manually entered command, such as a command issued by a user over the GUI 300 described in relation to Figure 3. For example, by tapping the SpO2 feedback portion 340 (e.g., when the clinician or other user feels that the indicated SpO2 level or trend is not good), the user may generate the trigger to initiate the learning procedure, effectively requesting that the controller 130 provide, on demand, a recommendation for adjusting the parameters of the therapy.

It should be noted that the trigger may comprise a combination of any such contemplated triggers. For example, an SpO2 threshold for triggering the learning procedure might be different depending on how much time has elapsed since the previous learning procedure was performed, thus allowing for the effect of a previous adjustment of the therapy parameters to be fully realized before initiating a new learning procedure. In this case, the trigger may comprise both a length of time and an SpO2 measurement, for example, as necessary conditions defining the trigger. As another example, an elapsed period of time and/or SpO2 or other sensor measurement may cause the system 100 to generate an audible or visual alarm (e.g., flashing SpO2 feedback portion 340), prompting the user to input a manual command to initiate the learning procedure (e.g., by tapping the SpO2 feedback portion 340, pressing a button, etc.). In this case, the trigger may comprise a time-based or measurement-based trigger and a manually entered command, for example. It is also contemplated that more than one of these various triggers (including those based on multiple criteria) may be supported by the system 100 and may, for example, be selectable and/or modifiable (e.g., changing a trigger threshold, period, etc.) at the option of the user or provider of the system 100. It should also be noted that the trigger received by the controller 130 may be both generated and received by the controller 130. For example, the controller 130 may receive one or more inputs (e.g., sensor readings, time information, user input, etc.) and may identify a combination of such inputs as a trigger, such that the trigger might not necessarily have existed as such outside of the controller 130 yet may nevertheless be considered received by the controller 130. Along the same lines, a trigger may be identified/generated wholly internal to the controller 130, as may be the case when a time-based trigger is received by the controller 130 from an internal clock thereof.

The operational flow of Figure 5 may continue with executing the learning procedure in response to the trigger (step 520). For example, as exemplified by the sub-operational flow shown in Figure 6, the controller 130 may, in response to the trigger, vary a flow parameter (step 522), vary an O₂ concentration parameter (step 524), and vary a temperature parameter (step 526) in any order and/or combination(s). On the basis of resulting pulse oximeter measurement(s), the controller 130 may determine one or more of these parameters to be a recommended parameter for adjustment (step 528). Steps 522-528 may be performed as described above in relation to Figure 2, for example, with the controller 130 performing a plurality of experimental runs in which a parameter of the airflow source 112 (e.g., a first parameter), a parameter of the valve 115 (e.g., a second parameter), and a parameter of the heater 170 (e.g., a third parameter) are varied by small amounts individually and/or in various combinations and corresponding measurements of the pulse oximeter 140 are recorded. The measurements of the pulse oximeter 140 may then be compared to determine, as the recommended parameter(s), the most influential parameter(s) on the patient's SpO2 (or the most efficient parameter to adjust for modifying the patient's SpO2).

It is also contemplated that the controller 130 may use a machine learning algorithm to determine the recommended parameter(s), which may in some cases include communicating with a third-party machine learning platform (e.g., over the Internet). For example, in order to execute the learning procedure, the controller 130 may apply a machine learning model to a dataset comprising the small variations of parameters and resulting SpO2 readings, with the output of the machine learning model being the recommended parameter(s). Such a machine learning model may be trained using historical datasets collected by the controller 130, for example, where each historical datasets may comprise i) the small variations of parameters and resulting SpO2 readings, ii) the actual adjustment subsequently made to one or more parameters (e.g., manually by a clinician), and iii) the actual effect on the SpO2, depletion of O₂ gas, power usage, etc. caused by the adjustment. By training a machine learning model with this kind of training data, the machine learning model may recognize, for a given combination of variations/readings, which adjustments are the most effective and/or efficient.

Referring back to Figure 5, the operational flow may continue with outputting a recommendation to adjust the recommended parameter that was determined by the learning procedure (step 530). For example, the controller 130 may output the recommendation as a visual indication on the display 150 such as a visual message (e.g., graphics, text, etc.), as an audio message (e.g., speech, alarm tone, etc.), as a dataset (e.g., log data for auditing/analytics, training data for a machine learning model, etc.), as a command signal to an external device, etc., or any combination thereof. Referring to the exemplary GUI 300 shown in Figure 3, the recommendation may be output as a visual indication to one or more of the flow control portion 310, FiO2 control portion 320, and temperature control portion 330 thereof. For example, the controller 130 might cause the corresponding control tool 312, 322, 332 and/or display 314, 324, 334 of the recommended parameter to change color, become lit up, or otherwise display some indication that a manual adjustment to that parameter is recommended.

The recommendation output by the controller 130 may comprise, in addition to an indication of which parameter is recommended to be adjusted, a direction in which to adjust the recommended parameter. For example, continuing to refer to the exemplary GUI 300 of Figure 3, an arrow or other directional indicator may be displayed next to the display 314, 324, 334 of the relevant parameter to show whether the parameter should be increased or decreased. The recommendation may further comprise an amount by which to adjust the recommended parameter. As one contemplated example, the control tool 312, 322, 332 of the recommended parameter may be updated to visually show the recommended new position of the control tool 312, 322, 332 as a shadowed or highlighted portion. For example, in order to indicate that first parameter should be modified to bring the total flow from 45 LPM up to 50 LPM, the new position on the slider or other flow control tool 312 may be marked, highlighted, or otherwise clearly indicated, such that the user is able to simply slide the slider to the indicated position to achieve the recommended adjustment (thus also causing the total flow display 314 to be updated to the new value of 50 LPM). In this way, the GUI 300 may be used to output the recommendation simply and intuitively.

With the recommendation having been outputted as described above, the operational flow of Figure 5 may continue with receiving a user input to adjust one or more parameters of the therapy (step 540). For example, the controller 130 may receive a user input over the GUI 300 via touchscreen functionality of the display 150 or buttons, etc. as described above or by any other input method (e.g., via voice commands). In some cases, the GUI 300 or other user interface may be presented on a website accessible via a web browser or on a mobile application installed on the user's smartphone or other mobile device, such that the user may input an adjustment to the therapy on a device external to the system 100. In this regard, the user input may in some cases be received by the controller 130 by short-range wireless communication (e.g., Bluetooth), over a network such as a WiFi network and/or the Internet, etc.

The operational flow of Figure 5 may conclude with adjusting the recommended parameter (step 550). For example, the controller 130 may adjust the first parameter of the airflow source 112, the second parameter of the valve 115 connected to the oxygen gas source, and/or the third parameter of the heater 170 to affect the adjustments in accordance with the user input received in step 540. For example, in order to increase the total flow (e.g., from 45 LPM to 50 LPM), the controller 130 may increase a blower speed or otherwise adjust the airflow source 112, possibly also adjusting one or more of the other parameters to the extent that the total flow is codependent on more than one parameter (e.g., adjusting the valve 115 to slightly increase the flow of O2 gas to maintain the same specified FiO2 in light of the increased quantity of ambient air entering the blender 111). In a case where the controller 130 acts autonomously without manual input by a clinician or other user, steps 530 and 540 may be completely omitted, in which case step 550 may proceed directly after step 520 with the controller 130 adjusting the recommended parameter automatically. In either case, the operational flow of Figure 5 may allow for more efficient and more effective adjustments to the high-flow respiration therapy administered to the patient using the system 100 while reducing the likelihood of clinician error.

As noted above, the controller 130 may in some embodiments act autonomously to automatically adjust recommended parameters. In this regard, it is contemplated that the high-flow respiratory therapy system 100 may be switchable between a clinician-controlled mode (including steps 530 and 540 of Figure 5) and an autonomous mode (omitting steps 530 and 540). Since the autonomous mode may allow the controller 130 to freely adjust parameters of the therapy to change the patient's SpO2, it may be possible for the controller 130 to maintain the SpO2 at a desired level through continual adjustments. As such, the GUI 300 of Figure 3 may be modified to allow the SpO2 to be set directly for closed-loop feedback control of the patient's oxygen saturation, rather than merely displayed as a result of the other settings (such as FiO2). When in the autonomous (closed-loop) mode, the GUI 300 may, for example, replace the FiO2 control portion 320 with an SpO2 control portion, allowing the user to adjust a set point of the patient's SpO2, which may then be effectively and efficiently maintained by the controller 130 using the learning procedure described herein.

The controller 130 of the high-flow respiratory therapy system 100 may be implemented with a programmable integrated circuit device such as a microcontroller or control processor, which may include one or more I/O and/or network interfaces for communication with external devices as described above. Broadly, the device may receive certain inputs, and based upon those inputs, may generate certain outputs. The specific operations that are performed on the inputs may be programmed as instructions that are executed by the control processor. In this regard, the device may include an arithmetic/logic unit (ALU), various registers, and input/output ports. External memory such as EEPROM (electrically erasable/programmable read only memory) may be connected to the device for permanent storage and retrieval of program instructions, and there may also be an internal random-access memory (RAM). Computer programs (e.g., software algorithms) for implementing any of the disclosed functionality of the controller 130, including the learning procedure described herein, may reside on such non-transitory program storage media, as well as on removable non-transitory program storage media such as a semiconductor memory (e.g. IC card), for example, in the case of providing an update to an existing device. Examples of program instructions stored on a program storage medium or computer-readable medium may include, in addition to code executable by a processor, state information for execution by programmable circuitry such as a field-programmable gate arrays (FPGA) or programmable logic device (PLD).

Embodiments of the invention can be described with reference to the following numbered clauses, with preferred features laid out in the dependent clauses:
1. A high-flow respiratory therapy system comprising:
   a blender arranged to receive a first gas and a second gas and to output a combination of the first gas and the second gas as a delivered gas to a patient respiratory interface;
   an airflow source for providing a flow of air to the blender as the first gas;
   a valve operable to provide oxygen gas from an oxygen gas source to the blender as the second gas;
   a heater operable to heat the delivered gas at the patient respiratory interface;
   a pulse oximeter; and
   a controller configured to execute a learning procedure in response to a trigger, the learning procedure comprising varying a first parameter of the airflow source, a second parameter of the valve, and a third parameter of the heater and determining a recommended parameter from among the first, second, and third parameters based on one or more measurements of the pulse oximeter, the controller further configured to output a recommendation to adjust the recommended parameter.
2. The high-flow respiratory therapy system of clause 1, wherein said varying the first parameter, the second parameter, and the third parameter includes performing a series of experimental runs, each of the runs including varying one or more of the first, second, and third parameters and recording a resulting measurement of the pulse oximeter.
3. The high-flow respiratory therapy system of clause 2, wherein said determining the recommended parameter includes comparing the recorded measurements of the pulse oximeter.
4. The high-flow respiratory therapy system of any preceding clause, wherein the trigger comprises a passage of a predefined length of time.
5. The high-flow respiratory therapy system of clause 4, wherein the trigger occurs periodically according to the predefined length of time.
6. The high-flow respiratory therapy system of any preceding clause, wherein the trigger comprises a predefined measurement of the pulse oximeter.
7. The high-flow respiratory therapy system of any preceding clause, wherein the trigger comprises a predefined degree of change in a measurement of the pulse oximeter.
8. The high-flow respiratory therapy system of any preceding clause, wherein the trigger comprises a manually entered command.
9. The high-flow respiratory therapy system of any preceding clause, wherein the controller is configured to output the recommendation as a visual indication on a display.
10. The high-flow respiratory therapy system of any preceding clasue, wherein the recommendation comprises a direction in which to adjust the recommended parameter.
11. The high-flow respiratory therapy system of clause 10, wherein the recommendation comprises an amount by which to adjust the recommended parameter.
12. The high-flow respiratory therapy system of any preceding clasue, further comprising:
   a flow sensor arranged to measure a flow rate of the delivered gas;
   an oxygen sensor arranged to measure a fraction of inspired oxygen (FiO2) of the delivered gas; and
   a temperature sensor arranged to measure a temperature of the delivered gas at the patient respiratory interface.
13. The high-flow respiratory therapy system of any preceding clause, further comprising a humidification system for humidifying the delivered gas as it flows from the blender to the patient respiratory interface.
14. The high-flow respiratory therapy system of clause 13, further comprising a second heater operable to heat the delivered gas upstream of the humidification system.
15. The high-flow respiratory therapy system of any preceding clause, wherein the airflow source comprises a blower or a compressed gas source.

## Claims

1. A method of controlling a high-flow respiratory therapy system, the method comprising:
receiving a trigger; and
executing a learning procedure in response to the trigger, the learning procedure comprising:
varying a first parameter of an airflow source that provides a flow of air to a blender of the high-flow respiratory therapy system;
varying a second parameter of a valve operable to provide oxygen gas from an oxygen gas source to the blender, the blender being arranged to receive the flow of air from the airflow source as a first gas, receive the oxygen gas from the valve as the second gas, and output a combination of the first gas and the second gas as a delivered gas to a patient respiratory interface;
varying a third parameter of a heater operable to heat the delivered gas at the patient respiratory interface; and
determining a recommended parameter from among the first, second, and third parameters based on one or more measurements of a pulse oximeter,
wherein the method further comprises outputting a recommendation to adjust the recommended parameter.

2. The method of claim 1, wherein said executing the learning procedure further comprises performing a series of experimental runs, each of the runs including varying one or more of the first, second, and third parameters and recording a resulting measurement of the pulse oximeter.

3. The method of claim 2, wherein said determining the recommended parameter includes comparing the recorded measurements of the pulse oximeter.

4. The method of any preceding claim, wherein the trigger comprises a passage of a predefined length of time.

5. The method of claim 4, wherein the trigger occurs periodically according to the predefined length of time.

6. The method of any preceding claim, wherein the trigger comprises a predefined measurement of the pulse oximeter.

7. The method of any preceding claim, wherein the trigger comprises a predefined degree of change in a measurement of the pulse oximeter.

8. The method of any preceding claim, wherein the trigger comprises a manually entered command.

9. The method of any preceding claim, wherein the recommendation is output as a visual indication on a display.

10. The method of any preceding claim, wherein the recommendation comprises a direction in which to adjust the recommended parameter.

11. The method of claim 10, wherein the recommendation comprises an amount by which to adjust the recommended parameter.

12. The method of any preceding claim, further comprising humidifying the delivered gas as it flows from the blender to the patient respiratory interface.

13. The method of claim 12, further comprising heating the delivered gas upstream of said humidifying.

14. A method of providing high-flow respiratory therapy to a patient, the method comprising:
the method of claim 1, wherein the patient respiratory interface is connected to the patient and said outputting comprises presenting the recommendation on a graphical user interface;
receiving a user input to the graphical user interface; and
adjusting the recommended parameter in response to the user input.

15. A non-transitory program storage medium on which are stored instructions executable by a processor or programmable circuit to perform the method of any one of the preceding claims.
